(19) Europäisches Patentamt
European Patent Office
Office européen des brevets

(11) Publication number: **0 420 102 A2**

# (12) EUROPEAN PATENT APPLICATION

(21) Application number: **90118309.5**

(51) Int. Cl.5: **G01N 33/58**, G01N 33/577

(22) Date of filing: **24.09.90**

(30) Priority: **28.09.89 US 414177**

(43) Date of publication of application:
**03.04.91 Bulletin 91/14**

(84) Designated Contracting States:
**AT BE CH DE ES FR GB IT LI NL**

(71) Applicant: **ABBOTT LABORATORIES**
**CHAD-0377, AP6D/2, One Abbott Park Road**
**Abbott Park, Illinois 60064-3500(US)**

(72) Inventor: **Blonski, David R.**
**4212- 53RD. Avenue**
**Kenosha, Wisconsin 53142(US)**

Inventor: **Hawksworth, David J.**
**709 Lakeside Drive**
**Vernon Hills, Illinois 60061(US)**
Inventor: **Flentge, Charles A.**
**37338 Fairview Lane**
**Lake Villa, Illinois 60046(US)**
Inventor: **Pennington, Charles, Jr.**
**552 Pamcourt**
**Wheeling, Illinois 60090(US)**

(74) Representative: **Modiano, Guido et al**
**MODIANO, JOSIF, PISANTY & STAUB**
**Modiano & Associati Via Meravigli, 16**
**I-20123 Milano(IT)**

## (54) Stabilization of monoclonal antibody for use in fluorescent polarization techniques.

(57) A method for determining ligands in a sample is disclosed. The method involves mixing with the sample in which the ligand is to be determined a tracer having the formula of Fig. 1 of the attached drawings or a biologically acceptable salt of such a tracer, a monoclonal antibody, and glycerol added as a part of a solution of the monoclonal antibody in which the glycerol is present in an amount sufficient to increase the stability of the monoclonal antibody in the solution, and then determining the amount of tracer bound to the antibody by fluorescence polarization techniques as a measure of the amount of ligand in the sample. In Fig. 1 of the drawings R is a ligand or analog thereof having at least one common epitope with a ligand to be determined so that the ligand to be determined and the ligand or analog thereof of the tracer are both specifically recognizable by a given antibody, and N is an integer from one to ten. The monoclonal antibody used is one which is capable of specifically recognizing both the ligand to be determined and the tracer. Glycerol is used in the monoclonal antibody solution in an amount, usually from about 5 percent to about 20 percent, sufficient to increase the stability of the monoclonal antibody. The optimum glycerol concentration has been found to be about 10%.

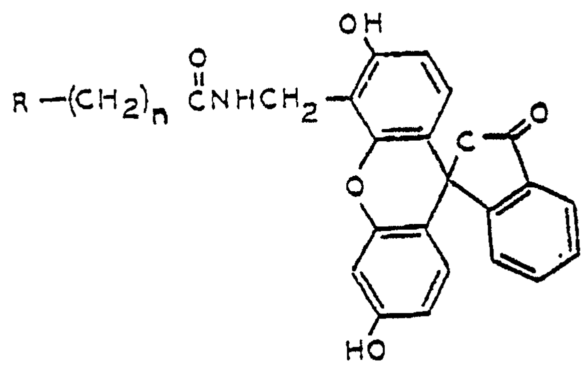

FIG. 1

EP 0 420 102 A2

# STABILIZATION OF MONOCLONAL ANTIBODY FOR USE IN FLUORESCENT POLARIZATION TECHNIQUES

## Field of the Invention

The invention is a method in which fluorescence polarization techniques are used for determining levels of biologically interesting moieties, e.g., ligands such as theophylline, in serum, plasma, urine and the like.

## Background of the Invention

The use of fluorescence polarization techniques for determining levels of biologically interesting moieties such as valproic acid in serum, plasma, urine and the like is known, being disclosed, for example, in U.S. patent No. 4,593,089, granted to Wang et al. on June 3, 1986. The patent discloses, by way of example, a method for determining levels of valproic acid in samples which involves mixing with the samples a biologically acceptable salt of 2-ethyl-5-amino-pentanoic acid-5-[(4,6-dichlorotriazin-2-yl)-amino]-fluorescein conjugate as a tracer, and sheep antiserum to valproic acid as an antibody which recognizes both the valproic acid and the tracer. The valproic acid (a ligand) and the tracer both form complexes with the antibody; the concentrations of tracer and of antibody are both kept constant so that the ratio of ligand-antibody complex to tracer-antibody complex that is formed is directly proportional to the amount of ligand present in the sample. Therefore, upon exciting the mixture with fluorescent light and measuring the polarization of the fluorescence emitted by the tracer and the tracer-antibody complex, it is possible to determine the amount of ligand in the sample by fluorescence polarization techniques.

Fluorescence polarization techniques, as is disclosed in the aforesaid Wang et al. patent, involve determining the polarization of fluorescence of several samples, some known and some unknown, preparing a standard curve showing polarization as a function for concentration from the data for the known samples, and then using the standard curve to determine the concentration of the unknown samples from the measured polarization. The patent also discloses that the method is operable to determine the concentrations of various ligands including steroids such as estrone, estradiol, cortisol, testosterone, progesterone, chenodeoxycholic acid, digoxin, cholic acid, digitoxin, deoxycholic acid, lithocholic acids and the ester and amide derivatives thereof; vitamins such as B-12, folic acid, thyroxine, triiodothyronine, histamine, serotonin, prostaglandins such as PGE, PFG, PGA; antiasthamatic drugs such as theophylline; antineoplastic drugs such as doxorubicin and methotrexate; antiarrhythmic drugs such as disopyramide, lidocaine, procainamide, propranolol, quinidine, N-acetyl procainamide; anticonvulsant drugs such as phenobarbital, phenytoin, primidone, valproic acid, carbamazepine and ethosuximide; antibiotics such as penicillins, cephalosporins, erythromycin, vancomycin, gentamicin, amikacin, chloramphenicol, streptomycin and tobramycin; antiarthritic drugs such as salicylate; antidepressant drugs including tricyclics such as nortriptyline, amitriptyline, imipramine and desipramine; and the like as well as the metabolites thereof.

It is also known that certain aminomethylfluoresceins can be used as tracers in using fluorescence polarization techniques to determine levels of biologically interesting moieties such as valproic acid in serum, plasma, urine and the like. By way of example, U.S. patent No. 4,510,251, granted to Kirkemo et al. on April 9, 1985, discloses a method for determining levels of estriol and of cortisol in samples which involves mixing with the samples estriol carboxymethyloxime aminomethylfluorescein and cortisol-3-carboxymethyloxime aminomethylfluorescein, respectively, as tracers, and antiserum raised against estriol and antiserum raised against cortisol, respectively, as antibodies, and then determining the amount of tracer-antibody conjugate by fluorescence polarization techniques. The Kirkemo et al. patent discloses that the ligands named above can be determined by the indicated method and, in addition, that drugs of abuse such as morphine, heroin, hydromophone, oxymorphone, metapon, codeine, hydrocodone, dihydrocodiene, dihydrohydroxy codeinone, pholcodine, dextromethorphan, phenazocine and deonin and their metabolites can be determined by the method.

## Summary of the Invention

The instant invention is based upon the discovery of a method that involves using fluorescence polarization techniques to determine various ligands in systems which include the ligand to be determined, a monoclonal antibody and one of certain members of a family of aminomethylfluorescein tracers, wherein

the stability of the monoclonal antibody in the system is unexpectedly and significantly increased by comparison with the stability of monoclonal antibodies in systems that have previously been used in making such determinations by including glycerol in the system. The ligands which can be determined by the present method include, but are not intended to be limited to, steroids such as estrone, estradiol, cortisol, testosterone, progesterone, chenodeoxycholic acid, digoxin, cholic acid, digitoxin, deoxycholic acid, lithocholic acids and the ester and amide derivatives thereof; vitamins such as B-12, folic acid, thyroxine, triiodothyronine, histamine, serotonin, prostaglandins such as PGE, PGF, PGA; antiasthamatic drugs such as theophylline; antineoplastic drugs such as doxorubicin and methotrexate; antiarrhythmic drugs such as disopyramide, lidocaine, procainamide, propranolol, quinidine, N-acetyl procainamide; anticonvulsant drugs such as phenobarbital, phenytoin, primidone, valproic acid, carbamazepine and ethosuximide; antibiotics such as penicillins, cephalosporins, erythromycin, vancomycin, gentamicin, amikacin, chloramphenicol, streptomycin and tobramycin; antiarthritic drugs such as salicylate; antidepressant drugs including tricyclics such as nortriptyline, amitriptyline, imipramine and disipramine; drugs of abuse such as morphine, heroin, hydromophone, oxymorphone, metapon, codeine, hydrocodone, dihydrocodiene, dihydrohydroxy codeinone, pholcodine, dextromethorphan, phenazocine and deonin and their metabolites. In particular, the invention is based upon the discovery that the stability of buffered solutions of monoclonal antibodies which are capable of recognizing the aminomethylfluorescein tracer is unexpectedly and significantly increased by the presence of from about 5 percent to about 20 percent, preferably from about 8 percent to about 12 percent, and most desirable about 10 percent of glycerol therein, and that the presence of glycerol does not interfere with the determinations in question.

Accordingly, in one aspect, the invention is a method for determining ligands in a sample which comprises mixing with the sample:

(a) a tracer having the formula of Fig. 1 of the attached drawings where R is a ligand or analog thereof having at least one common epitope with a ligand to be determined so that the ligand to be determined and the ligand or analog thereof of the tracer are both specifically recognizable by a given antibody and n is an integer from one to ten, or a biologically acceptable salt of such a tracer,

(b) a monoclonal antibody which is capable of specifically recognizing both the ligand to be determined and the tracer, and

(c) glycerol, and then determining the amount of tracer bound to the antibody by fluorescence polarization techniques as a measure of the amount of ligand in the sample. The glycerol present in a sample used in practicing the present invention is introduced as a part of a solution which includes the monoclonal antibody; the amount of glycerol in the antibody solution should be sufficient to increase the stability of the monoclonal antibody to a significant extent. It has been found that as little as 5 percent and as much as 20 percent of glycerol in the monoclonal antibody solution is effective to increase the stability of the monoclonal antibody significantly, but that the stability of the monoclonal antibody decreases when the solution contains as much as 30 percent of glycerol.

In another embodiement, the invention is a buffered solution of a monoclonal antibody which is capable of specifically recognizing both a ligand to be determined and a tracer having the formula of Fig. 1 of the attached drawings where R is a ligand or analog thereof, and n is an integer from 1 to 10, and an amount of glycerol sufficient to increase significantly the stability of the monoclonal antibody in the solution.

## Description of the Preferred Embodiments

The invention will be more fully understood from the following example, which constitutes the best mode presently contemplated by the inventor, but is presented solely for the purpose of illustration, and is not to be construed as limiting.

As used herein, and in the appended claims, the terms "percent" and "parts" refer to percent and parts by weight, unless otherwise indicated; g means gram or grams; mg means milligran or milligrams; ng means nanogram or nanograms; cm means centimeter or centimeters; mm means millimeter or millimeters; 1 means liter or liters; μl means microliter or microliters; $^{m}/_{o}$ means mole percent, and equals 100 times the number of moles of the constituent designated in a composition divided by the total number of moles in the composition; $^{v}/_{v}$ means percent by volume; M means molar and equals the number of moles of a solute in 1 liter of a solution; psi means pounds per square inch; and MPa means $10^6$ Pascals. All temperatures are in ° C., unless otherwise indicated.

Example

A theophylline-aminomethylfluorescein derivative having the structure of Fig. 1 of the drawings where n is 5 and R has the structure of Fig. 2 of the drawings was produced from 60 mg of 8-carboxypentyl theophylline dissolved in 4.0 ml tetrahydrofurane, 26.0 mg of N-hydroxysuccinimide, 46.0 mg of dicyclohexylcarbodiimide and 81.0 mg of aminomethylfluorescein (U.S. patent No. 4,614,823, Example 2). The N-hydroxy succinimide and 2 ml dimethylformamide were added to the 8-carboxypentyl theophylline, and reaction was allowed to proceed at room temperature of about 20° for three hours. The aminomethylfluorescein and 2 ml dimethylformamide were then added to the reaction mixture. Reaction was allowed to proceed to completion at room temperature, which required about 15 hours. The solvent was then removed under vacuum, leaving a crude product which was dissolved in a solvent composed of methylene chloride containing $15^{v}/_{v}$ methanol and purified by preparative thin-layer chromatography.

The theophylline-aminomethylfluorescein derivative produced as described above was then used as a tracer in fluorescence polarization immunoassays conducted as follows:

(1) a measured volume of a standard or test serum was delivered into a test tube and diluted with a buffer;

(2) a known concentration of a tracer of the instant invention was then added to each tube;

(3) a known concentration of an antiserum was added to the tubes;

(4) the reaction mixture was incubated at 35°; and

(5) the amount of tracer bound to monoclonal antibody was measured by fluorescence polarization techniques as a measure of the amount of ligand (serum) in the sample.

The following materials were used in carrying out the immunoassays:

1. The theophylline-aminomethylfluorescein tracer produced as described above in a 0.1M solution of a buffer (pH 7.5) which is commercially available under the trade designation "TRIS" containing 0.01% bovine gammaglobulin (BGG) and 0.1% sodium azide.

2. Anti-theophylline monoclonal antibody antiserum derived from mouse ascites in 10% aqueous glycerol containing 0.1% sodium azide, 1.0% chicken egg albumin hydrolysate, and 0.9% sodium chloride.

3. Pretreatment solution consisting of 2.5% 5-sulfosalicylate and 0.1M solution of TRIS.

4. Samples of human serum or other biological fluid containing theophylline.

5. Cuvettes, 10x75 mm glass culture tubes used as cuvettes.

6. Fluorometer capable of measuring fluorescence polarization with a precision of ± 0.001 unit.

The assay method involved the following steps:

1. A 21.6 µl sample was pipetted into a predilution container with 25 µl antiserum and 25 µl of pretreatment solution. The volume of the solution in the predilution container was diluted to approximately 500 µl with BGG buffer.

2. A 175 µl charge of the solution from the predilution container was pipetted into a cuvette and was diluted to a final volume of 0.99 ml with BGG buffer.

3. The contents of the cuvette were mixed well. After three minutes, a background determination of fluorescence polarization was made.

4. An additional 175 µl portion of the predilution mixture and a 25 µl portion of the tracer were added to the cuvette, and BGG buffer was charged to bring the total volume in the cuvette to about 2.0 ml.

5. The contents of the cuvette were mixed well and allowed to incubate for seven minutes at 35°.

6. The fluorescence polarization value of the composition in the cuvette was then determined using an appropriate instrument (fluorometer).

The results of a series of serum standards containing theophylline at concentrations between 0 and 40 ng per ml are set forth below:

| Concentration of theophylline, ng/ml | Polarization |
|---|---|
| 0.00 | 0.265 |
| 2.50 | 0.232 |
| 5.00 | 0.204 |
| 10.00 | 0.162 |
| 20.00 | 0.116 |
| 40.00 | 0.079 |

To investigate stability at various concentrations of glycerol, anti-theophylline monoclonal antibody antiserum derived from mouse ascites in 10% aqueous glycerol containing 0.1% sodium azide, 1.0% chicken egg albumin hydrolysate, and 0.9% sodium chloride ("10% glycerol") was prepared, as was another antiserum which was identical except that it contained 20 percent of glycerol ("20% Glycerol"). These antiserums were then used to assay a serum theophylline sample by carrying out steps 1 through 6 of the assay method described above:

(a) using the freshly prepared sample

(b) using samples that had stood for varying periods of time at 45°, and

(c) using samples that had stood for varying periods of time at 2 to 8°. The polarization found using each of the two serum theophylline samples, when freshly prepared, after standing for 7 days at 45°, and after standing for 2 months at 2-8° are set forth in the following table:

| | Freshly prepared | After 7 days at 45° | After 2 months at 2-8° |
|---|---|---|---|
| 10% Glycerol | 208.73 | 199.04 | 204.46 |
| 20% Glycerol | 201.97 | 195.45 | 197.88 |

The foregoing data indicate satisfactory stability of the two serum theophylline samples. When, however, for purposes of comparison and not in accordance with the present invention, samples containing 30 percent of glycerol in one case and no glycerol in another case, but otherwise identical, are subjected to the foregoing test for stability, significantly greater variations in the assays are encountered and the stability is deemed to be unsatisfactory.

It will be appreciated that other theophylline-aminomethylfluorescein tracers for use in practicing the method of the instant invention can be produced by the method of the foregoing example by substituting equivalent amounts of other 8-carboxyalkyl theophyllines for the 8-carboxypentyl theophylline of the example, and that other ligandaminomethyl-fluorescein tracers can be so produced by so substituting equivalent amounts of other carboxyalkyl ligands. Similarly, carboxyalkyl ligand analogs can be so substituted to produce tracers which are ligand analog-aminomethylfluoresceins. In a like manner, the other ligand-aminomethylfluorescein and ligand analogaminomethylfluorescein tracers and biologically acceptable salts of all such tracers can be used in the procedure described in the foregoing example with appropriate monoclonal antibodies to determine other ligands. The ligands to be determined and the ligand or ligand analog of the tracer must have at least one common epitope, and the monoclonal antibody must be capable of specifically recognizing both the ligand and the tracer.

It will be appreciated that, while only preferred embodiments thereof have been described, various changes and modifications are possible without departing from the spirit and scope of the invention as defined in the appended claims.

## Claims

1. A method for determining ligands in a sample, characterized in that said method comprises mixing with the sample:

(a) a tracer having the following formula

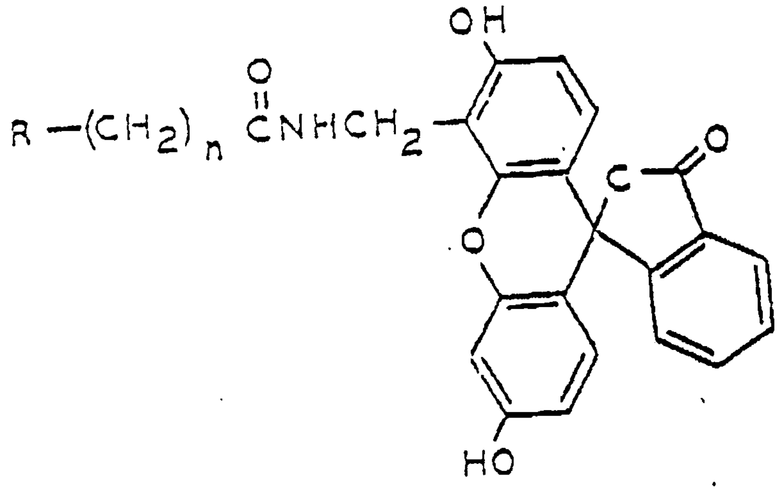

where R is a ligand or analog thereof having at least one common epitope with a ligand to be determined so that the ligand to be determined and the ligand or analog thereof of the tracer are both specifically recognizable by a given antibody, and n is an integer from one to ten, or a biologically acceptable salt of such a tracer,

(b) a monoclonal antibody which is capable of specifically recognizing both the ligand to be determined and the tracer, and

(c) glycerol added as a part of a solution of the monoclonal antibody in which the glycerol is present in an amount sufficient to increase the stability of the monoclonal antibody in the solution, and then determining the amount of tracer bound to the antibody by fluorescence polarization techniques as a measure of the amount of ligand in the sample.

2. A method as claimed in claim 1 characterized in that the glycerol is present in the monoclonal antibody solution in an amount ranging from about 5% to about 20%.

3. A method as claimed in claim 1 characterized in that the glycerol constitutes about 10% of the monoclonal antibody solution that is added to the sample subjected to fluorescence polarization techniques.

4. A method as claimed in claim 1 characterized in that R has the following structure

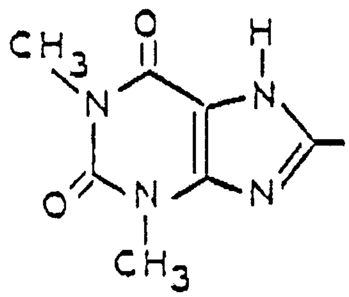

5. A method as claimed in claim 4 characterized in that the glycerol is present in the monoclonal antibody solution in an amount ranging from about 5% to about 20%.

6. A method as claimed in claim 4 characterized in that the glycerol constitutes about 10% of the monoclonal antibody solution that is added to the sample subjected to fluorescence polarization techniques.

7. A buffered solution of a monoclonal antibody which is capable of specifically recognizing both a ligand to be determined and a tracer having the formula

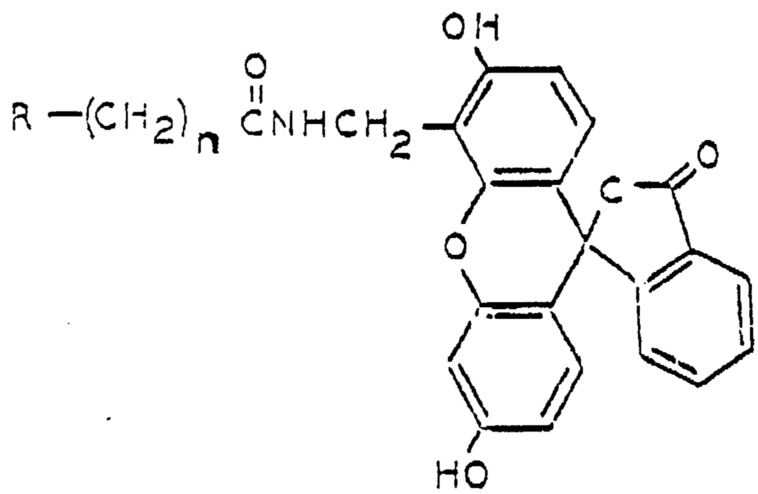

where R is a ligand or analog thereof, and n is an integer from 1 to 10, and an amount of glycerol sufficient to increase significantly the stability of the monoclonal antibody in the solution.

8. A solution as claimed in claim 7 characterized in that the glycerol constitutes from about 5% to about 20% of the solution.

9. A solution as claimed in claim 7 characterized in that the glycerol constitutes from about 8% to about 12% of the solution.

10. A solution as claimed in claim 7 characterized in that the glycerol constitutes about 10% of the solution.

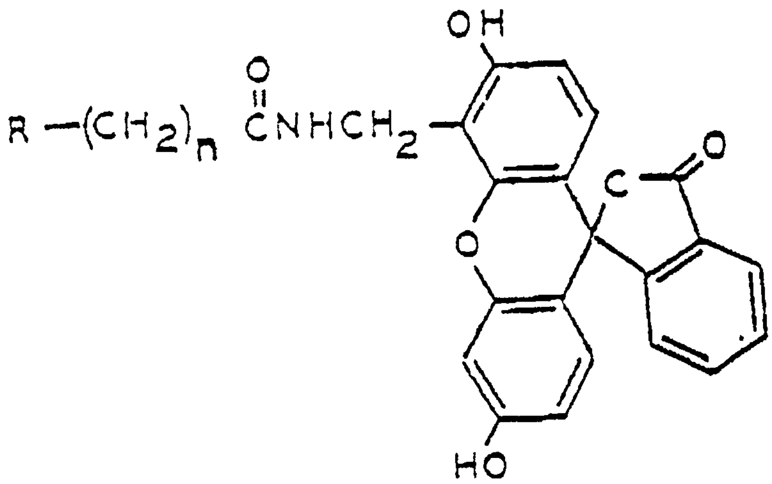

FIG. 1

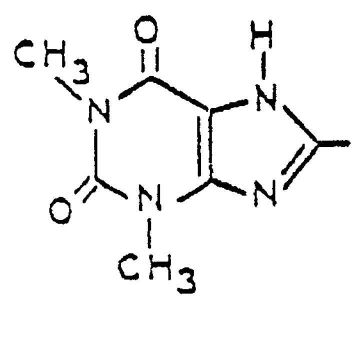

FIG. 2